# EUROPEAN PATENT SPECIFICATION

(11) **EP 0 953 366 B1**
(45) Date of publication and mention of the grant of the patent: **23.06.2004**
(21) Application number: 97944151.6
(22) Date of filing: 15.10.1997
(51) Int. Cl.: A61N 1/00, A61N 1/20, A61N 1/24

(54) **THERAPEUTICAL DEVICE EQUIPPED WITH TAPE**
MIT EINEM BAND VERSEHENE THERAPEUTISCHE VORRICHTUNG
DISPOSITIF THERAPEUTIQUE EQUIPE D'UNE BANDE

(30) Priority: 16.10.1996 JP 29568096
(43) Date of publication of application: 03.11.1999
(73) Proprietor: Kabushiki Kaisha Sun Field, Hakata-ku, Fukuoka-shi, Fukuoka 812 (JP)
(72) Inventor: YAMAGUCHI, Yoshimi, Kabushiki Kaisha Sun Field, Hakata-ku Fukuoka-shi Fukuoka 812 (JP); MORIYAMA, Hiroshi, Kabushiki Kaisha Sun Field, Hakata-ku Fukuoka-shi Fukuoka 812 (JP)
(74) Representative: Müller-Boré & Partner Patentanwälte
(86) International application number: PCT/JP1997/003747
(87) International publication number: WO 1998/016276

(56) References cited:
- FR-A- 2 626 181
- JP-A- 5 154 205
- JP-U- 5 051 340
- JP-U- 58 131 836
- US-A- 4 633 888

## Description

### TECHNICAL FIELD

The present invention relates to a therapeutical device in which with respect to the morbid condition of a disease such as stiffness or a pain of muscles of the human body, a living body current and a living body electromagnetic wave of the affected part are collected and abstracted, and a current flowing therethrough is commutated in a unidirectional and electrically short-circuited to allow the disease take a favorable turn, and a therapeutical device with a tape.

### BACKGROUND ART

Conventionally known means for curing stiffness or a pain of various parts of the human body include a stupe, a massage, acupuncture and so on, all of which attempt to quicken the circulation of the blood so as to lighten the disease.

There is a further method for applying to the affected part metallic particles which generate a magnetic force of more or less 800 gauss. In this method, the magnetic force stimulates the affected part to impart a magnetic field to a flow of ion in blood to make the circulation of blood smooth.

There are a variety of causes of the poor circulation of blood, one of which is an abnormality of a living body current accompanied by an electromagnetic wave generated from the living body.

Therefore, as means for improving the abnormality of the living body current, a living body current commutation therapeutical method is known. This stiffness or a pain of muscles of the human body living body current commutation therapeutical method makes use of a commutation action of a current of an electron element, in which an electron element having such characteristics as described is applied to the body surface of a patient, and a disturbance of a living body current is put in order by making use of the current characteristics to improve the circulation of blood. The therapeutical effect has been considered clinically.

However, in the means for curing stiffness or a pain of various parts of the human body by a stupe, a massage, acupuncture and so on, in most cases, the poor circulation of blood canbe improved, but a dominant cause of generating the poor circulation of blood itself cannot be removed. Accordingly, this has the therapeutical effect, but it requires a long period of time till the effect is obtained, and in addition if the therapy is stopped, the disease possibly reoccurs.

Further, the method for applying the magnetism to the affected part also poses a problem in that it takes a little time till the effect appears, and when the magnetism is strengthened, the sub-action of a poor physical condition such as a burden imposed on the heart or the like occurs.

Furthermore, the method for applying an electron element to the affected part has a problem in that depending on the operating procedure requiring other techniques such that the electron element is applied so that polarities are arranged on the extreme ends of legs or the heart, there is a great difference in appearance ofthe effect, and therefore, in use of individuals, its use is immoderate; and metal parts on the surface of an electron element are soldered so that application and contact thereof to the skin for a long period of time induces allergy of the skin.

Document JP-U-5154205 shows a therapeutic device comprising a unidirectional current commutator, e.g. a diode, having a pair of poles connected to a current connecting probe, whereby said current collecting probe consists of an electric conductor. Said device is applied to the skin of a human body, to which it can be temporarily attached by a breathable tape.

Document JP-U-58131836 shows a therapeutic device comprising a diode having a pair of poles, whereby said diode is attached to an adhesive magnetic sheet comprising an opening. Said magnetic sheet can be attached to the skin of a human body, thereby fixing the diode to on the surface of the skin such that said the poles of said diode establish electrical contact with the skin, whereby the diode is located inside the opening of the magnetic sheet.

Document JP-U-5051340 shows a therapeutic device comprising a pushing element, which can be attached to human skin by means of an adhesive sheet, which comprises a plurality of pin holes for ventilation.

It is the object of the invention to provide a therapeutic device, which can be applied safely and easily and which can expect a wide range of therapeutic effects.

This object is fulfillled by a therapeutic device having the features disclosed in claim 1. Preferred embodiments are defined in the dependent subclaims.

A therapeutical device with an adhesive tape according to the invention comprises a unidirectional current commutator, a current collection probe and a tape, wherein said current collection probe formed from a copper wire extending from both polarity ends of said commutator and being provided around said commutator in a substantially circular shape in the periphery of an outer extending portion derived from both ends thereof, said wires being wound around said commutator by 3/4 turn in a direction opposite to each other, said wires having ends bent internally toward said unidirectional current commutator, wherein said current commutator is located in the center and a current flows in said wires therearound to form a magnetic flux, and the current collection probe having its outer peripheral surface applied with a plating of one metal, which is an electrical good conductor, out of gold, titanium, platinum and silver, wherein said tape, having a tackiness on one surface thereof, is applied hard to the skin surface of the affected part from one surface in which the unidirectional current commutator and the current collection probe are integrated, so that the integrated surface is applied to the skin surface, wherein a protruding portion is provided on a side of said unidirectional current commutator so as to be placed in contact with the skin surface and said tape is provided with a vent hole having the size to the extent, that a part or the whole is exposed to the unidirectional current commutator while being deviated from the surface thereof in a central portion of the tape and is provided with a plurality of pores spaced apart from each other.

The therapeutical device according to the present intention has an outer peripheral surface of the current collection probe applied with plating of either metal, which is an electric good conductor, out of gold, silver, titanium, platinum and so on which creates no unusual change such as skin eruptions resulting from contact for a long period of time on the skin surface.

### BRIEF DESCRIPTION OF THE DRAWINGS

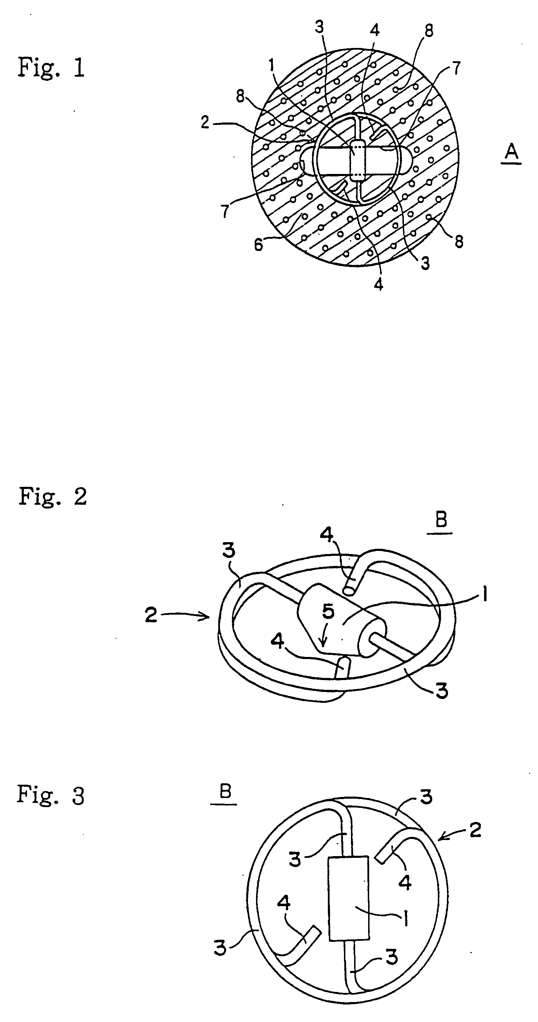

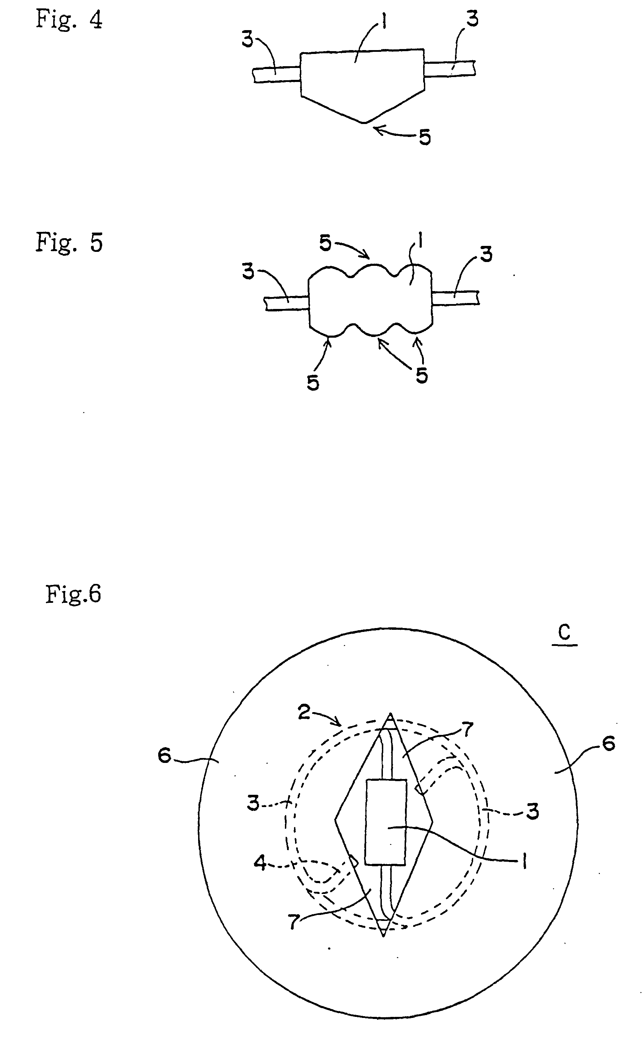

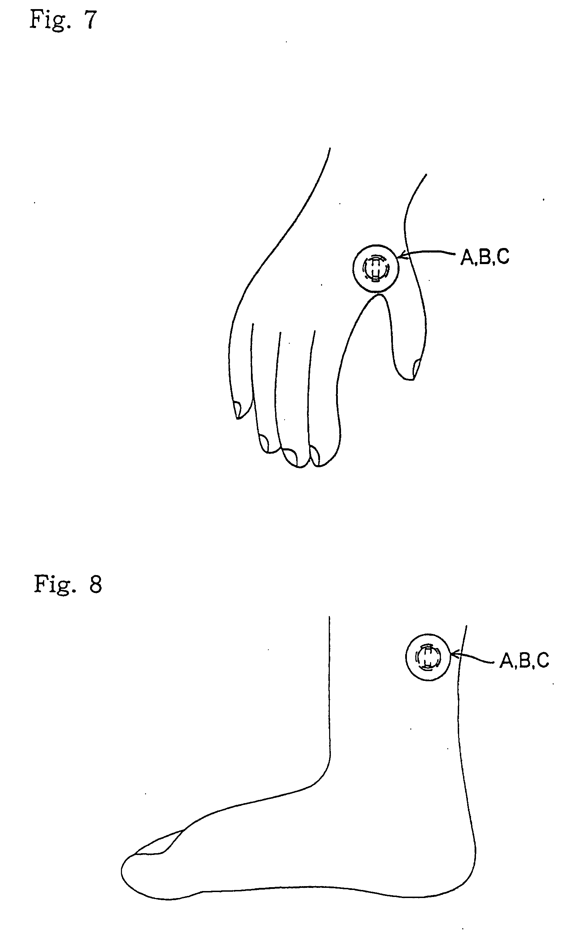

FIG. 1 is a plan view showing a therapeutical device with a tape according to a second embodiment of the present invention; FIG. 2 is a perspective view showing a therapeutical device according to a third embodiment of the present invention in which a protruding portion is provided on a unidirectional current commutator; FIG. 3 is a plan view showing a therapeutical device according to the third embodiment of the present invention which is provided with a protruding portion; FIG. 4 is a side view showing a protruding portion provided on a unidirectional current commutator according to the third embodiment of the present invention; FIG. 5 is a side view showing a protruding portion of a different shape provided on a unidirectional current commutator according to a fourth embodiment ofthe present invention; FIG. 6 is a plan view showing a therapeutical device with a tape according to a fifth embodiment of the present invention; FIG. 7 is an explanatory view showing a state in which the therapeutical device according to the present invention is mounted on the joint of the thumb and the index finger of the human body; and FIG. 8 is an explanatory view showing a state in which the therapeutical device according to the present invention is mounted at the rear of an ankle of the human body.

### BEST MODE OF CARRYING OUT THE INVENTION

First, the principle of the present invention will be mentioned.

The present inventor has found that it is effective for the therapy of the affected part to collet as much as living body currents, while in the prior art, importance is merely attached to a direction of polarity. That is, in the prior art, the theme merely comprises a commutation of a living body current, and also in the art of this kind, it has been excessively attached that the device is applied to the affected part without mistaking the polarities.

However, the present inventor has assumed that in the living body current commutation therapeutical method of this kind, the therapeutical efficiency would be enhanced rather by an amount of living body currents collected from the human body.

A cell of the human body is considered as one kind of a battery. That is, when a change in sound, heat, light and blood, a change in temperature or the like is applied to the human body, this constitutes a stimulus to urge the cell activation and generate a living body current and a living body electromagnetic wave.

Generally, a single cell voltage in the healthy time is - 90 mV, and when receiving an external stimulus, it rises to approximately +110 mV instantaneously (2/10 sec), and after this, it returns to an original state. That is, a voltage variation of +110 mV is generated.

Grasping it by a group of cells, when a voltage variation of 110 mV occurs in a specific cell, it is transmitted to an adjacent cell. Therefore, when 10 cells are totaled, +1100 mV, i.e., abnormal voltage of 1.1V occurs. It is contemplated that this abnormal voltage comprises the cause of stiff shoulders and a lumbago.

That is, the generation of the abnormal voltage by the defective circulation of blood renders the therapy of the affected part possible.

The present inventors therefore have found that the abnormal voltage is collected as much as possible from the group of cells to enhance the therapeutical effect. Further, the therapeutical device is applied to the affected part, whereby the affected part can be applied hard to erase the abnormal voltage and apply the affected part hard to lighten the stagnation of the circulation of the blood. Further, when the device is applied to the affected part in a state with a unidirectional current commutator exposed, whereby a ray of light is accumulated in the therapeutical device to create heat in the therapeutical device, thus obtaining the hot acupuncture effect. By the hot acupuncture effect a living body current can be activated, and the commutation effect of the therapeutic device can be enhanced.

The respective embodiments ofthe present invention will be described in detail hereinafter with reference to the accompanying drawings.

In all the drawings, the same reference numerals indicate the same or corresponding members.

### Embodiment 1

FIG. 1 is a plan view showing a therapeutical device according to a first embodiment of the present invention;

That is, first, there is provided the unidirectional current commutator 1 which is small elongated circular in the central part and filled with a semiconductor, and copper wires 3 are derived from both ends thereof, the two copper wires 3 being wound around the unidirectional current commutator 1 by about 3/4 turn in a direction opposite to each other, the two copper wires 3 having ends bended internally toward the unidirectional current commutator 1 to form the current collection probe 2, whereby the main body of the therapeutical device A is constituted.

Further, the surface of the unidirectional commutator 1 is applied with plating of metal out of gold, silver, titanium, and platinum. The steps of plating comprises degreasing - acid activity - neutralizing - plating - discolor preventing - finishing in the order as described, and water washing is required between the respective steps. Since the commutator is weak in acid, care should be taken in the operation of the acid activity and the like that industrial acid not influencing on the commutator be used. In the prior art, a plating method has been employed so as to pay attention to the commutator. However, if in a state in which the unidirectional commutator is removed, plating is applied merely to the current collection probe 2, and the unidirectional current commutator 1 is mounted after plating, a wide range of plating becomes enabled, and plating with high efficiency and high therapeutical effect can be applied.

The ends 4 of the copper wire 3 wound around are bended internally and reach the unidirectional current commutator 1 or reach close to the unidirectional current commutator 1. Since the copper wire 3 is bended, a collection area of a living body current is wide.

Further, the unidirectional current commutator 1 is located in the center, and a current flows into the copper wire 3 therearound to form a magnetic flux , which operation enhances the commutation effect, and the therapeutical effect can be enhanced.

The shape of the current collection probe 2 is optional, which includes, other than a circle, a square, a hexagon, an octagon, a decagon, a dodecagon and so on.

The unidirectional current commutator 1 is an element which is provided with a current commutation operation in which a semiconductor formed of silicon (Si) or germanium is subjected to spot junction or PN junction to thereby allow only a current from one terminal (+) to the other terminal (-), and is hermetically sealed by a cylindrical glass package. With this, the heat resistance can be enhanced by the glass package, and the unidirectional current commutator 1 is prevented from being watered.

Further, a tape 6 is formed to be circular, only one surface of which has a tackiness, the outer periphery of the tape surface is formed to be somewhat larger than that of the therapeutical device, and a square vent hole 7 is provided in the center of the tape surface.

The vent hole 7 has the size to the extent that a part or the whole of the unidirectional current commutator 1 is exposed, and has an elongated shape so as to cross perpendicular to the central portion of the unidirectional current commutator 1. By the provision of the vent hole 7, the ion action and the potential action in the tissue of the unidirectional current commutator 1 is active whereby the commutation effect is enhanced, and an itch or skin eruptions are hard to occur in a portion where the device is applied.

The tape 6 which is a support for the therapeutical device comprises, for example, an adhesive tape in which a tackifier formed of acrylic ester is uniformly coated on the skin-colored expansion plaster, which is an applying plaster with high safety.

The therapeutical device according to the first embodiment of the present invention was actually applied to inpatients and outpatients in a rehabilitation station of FUKUOKA UNIVERSITY hospital for the therapeutical experiment, details and results of which are mentioned and the effects of the present invention will be explained hereinafter.

A sample used comprises, as a shell, a cylindrical unidirectional current commutator 1 formed from a water-proof and pressure resistance unidirectional commutator 1, a circular current collection probe 2 in which a copper wire 3 is applied with gold plating, and a tape 6 (a plaster) which is applied hard to the affected part and one side of which is tacky and having a vent hole 7 bored in a central portion thereof, which constitution is as shown in Fig. 1.

The device was applied to the part to be a point of the patient under the instructions of a doctor, and a therapeutical device comprising a unidirectional current commutator 1 and a circular current collection probe 2 whose diameter is 9 mm and a diameter of a copper wire 3 is 0.5 mm was used. The observation and expression (VAS = visual analog scale and therapy are jointly used) of a change in a pain caused by stiffness an a self-conscious pain were conducted after the suitable passage of time before and after application, and the results thereof were examined.

For the purpose of comparison, the unidirectional current commutator 1 was not purposely connected, and a comparative device having a member with a portion thereof short-circuited by a copper wire (merely comprising a current collection probe 2 short-circuited by a copper wire and a tape 6) was used for the affected part of some patients. A difference from the present invention was observed.

As the standards of diseases, there were used and defined a slight degree (a stiffness and a pain are almost improved by a general therapeutic device (magnetism, low frequency, acupuncture, etc.), a middle degree (a stiffness and a pain are not sometimes improved by a general therapeutical device), and a serious degree (a stiffness and a pain are not at all improved by a general therapeutical device).

Further, the effectual effects were determined through the therapy including the VAS system as follows:
Max. Effect: Improvement achieved within 24 to 48 hours, or maximum value obtained within 72 hours by VAS inspection
Effective: Effect when improvement is made within 24 to 72 hours
Somewhat effective: Effect when improvement is made in not less than 72 hours
Ineffective: No improvement made
Stop: Drop out
Aggravation: Serious conditions of a disease

The judgement time set to 24 to 72 hours as a reference is based on consideration of an outpatient, and an idea that such a period oftime as described would be required on the average to confirm the effect of the therapeutical device with a tape according to the present invention.

The results of the therapeutical experiments carried out on patients are as listed in Tables 1 to 3, which are results in the case where the therapeutical device with a tape according to the present invention is applied.

On the other hand, the results obtained using a comparative device merely comprising a current collection probe 2 short-circuited by a copper wire an a tape 6 are shown in Table 4. Since the effects shown in Table 4 are obviously different from those shown in Tables 1 to 3, the number of persons scheduled to subject to the therapeutical experiment is changed and reduced, and all the thereafter patients are subjected to the therapeutical experiment using the therapeutical device with a tape according to the present invention.

The results of judgement obtained by totaling the above are shown in Table 5. These are referred to as the first experimental results.

That is, it has been found from the above-described results that out of 82 persons who used the therapeutical device with a tape according to the present invention, 72 persons are those belonging to Max. Effect and Effective, and this result indicates 88% of effective rate with respect to the condition of a disease of stiffness.

In the following Tables:
Some-effect: somewhat effective
M: male
F: female

**TABLE I**

| Name of patient | Sex distinction | Age | Name of disease | Condition of disease | Number of applications | Time of applications (Hr) | Judgement of results | Side effects |
|---|---|---|---|---|---|---|---|---|
| Z.T. | M | 57 | After operation of right femur fracture | serious | 4 | 72 | some-effect | No |
| K.M. | F | 77 | Left cervical Neuralgia | middle | 4 | 48 | effective | No |
| Y.K. | F | 69 | Lumbar vertebra stricture | middle | 6 | 72 | effective | No |
| I.M. | M | 68 | Deformed lumbar vertebra | middle | 6 | 48 | max. effect | No |
| M.Y | F | 53 | Left cervical neuralgia | middle | 5 | 24 | max.effect | No |
| M.Y | F | 53 | Left upper arm cervical Inflamation | middle | 3 | 24 | max.effect | No |
| K.S. | F | 54 | Left hand arthralgy | serious | 4 | 48 | effective | No |
| O.I. | F | 55 | Right cervical neuralgia.right [zimei] | middle | 5 | 24 | max.effect | No |
| O.I. | F | 55 | Right foot arthralgy | middle | 3 | 24 | max.effect | No |
| Y.Y. | F | 55 | Right shoulder articular circumferential inflammation | middle | 2 | 24 | max.effect | No |
| O.K.. | M | 39 | Lumbergo | middle | 5 | 24 | effective | No |
| O.Y.. | F | 38 | Lumbergo | middle | 6 | 24 | max.effect | No |
| O.Y. | F | 38 | Both cervical neuralgia | middle | 6 | 24 | max.effect | No |
| I.Y. | F | 67 | Spinal osteoporosis | middle | 6 | 48 | effective | No |
| T.H. | F | 72 | Left foot articular inflammation fracture | middle | 3 | 48 | effective | No |
| T H.. | F | 72 | Spinal osteoporosis | middle | 6 | 48 | effective | No |
| T.H. | F | 72 | Left cervical neuralgia | middle | 6 | 48 | effective | No |
| Y.M. | M | 69 | Lumbar vertebra stricture | middle | 6 | 24 | max. effect | No |
| Y.M. | M | 69 | Both shoulder articular circumferential inflammation | middle | 4 | 48 effective No | | |
| O.T. | M | 77 | Left cervical cervical neuralgia Inflamation | slight | 3 | 24 | max.effect | No |
| OT. | M | 77 | Vertebra Stricture | slight | 6 | 24 | max.effect. | No |
| K.H. | F | 60 | Right tibial neuralgia | Serious | 3 | 48 | max.effect | No |
| K.K. | M | 19 | Both tibial neuralgia | middle | 6 | 48 | ineffective | No |
| K.K. | M | 59 | Deformed limbar vertebra | middle | 5 | 24 | max.effect | No |
| A.H. | M | 51 | Deformed lumbar vertebra | middle | 8 | 48 | effective | No |
| H.K.. | F | 39 | Both tibial neuralgia | middle | 4 | 48 | effective | No |
| Y.O. | M | 74 | Deformed lumbar vertebra | middle | 6 | 24 | max.effect | No |
| T.M. | F | 77 | Spinal osteoporosis | middle | 6 | 24 | max.effect. | No |
| A.H. | M | 63 | Left foot arthritis | middle | 3 | 24 | max.effect. | No |
| O.T. | F | 57 | Right upper arm cervical inflammation | Serious | 3 | 72 | ineffective | No |
| H.K. | F | 54 | / Lumbergo | middle | 4 | 48 | effective | No |
| T.H. | F | 74 | Deformed lumbar vertebra | middle | 6 | 48 | effective | No |
| N Y. | F | 25 | Both tibial neuralgia | middle | 6 | 48 | effective | No |
| H.M.. | M | 71 | Deformed lumbar vertebra | middle | 3 | 24 | max. effect. | No |
| F.T. | F | 64 | Both tibial neuralgia | middle | 6 | 48 | effective | No |
| F.S. | F | 63 | Lumbergo | middle | 4 | 24 | max.effect. | No |
| K.M. | M | 80 | Both tibial neuralgia | middle | 6 | 48 | effective | No |

**TABLE 2**

| Name of patient | Sex distinction | Age | Name of disease | Condition of disease | Number of applications | Time of applications (Hr) | Judgement of results | Side effects |
|---|---|---|---|---|---|---|---|---|
| I.K. | M | 70 | Left shoulder articular circumferential Inflammation | serious | 5 | 24 | some-effect | No |
| Y.J. | F | 49 | Lumbergo | middle | 6 | 48 | effective | No |
| T.S. | M | 65 | Deformed lumbar vertebra | middle | 6 | 24 | max.effect. | No |
| K.Y. | F | 64 | Deformed lumbar vertebra | middle | 6 | 48 | effective | No |
| L.M. | F | 63 | Lumbergo | middle | 6 | 24, | max.effect | No |
| L.M. | F | 63 | Right tibial neuralgia | middle | 3 | 24 | max.effect | No |
| G.H. | F | 30 | Left tibial neuralgia | middle | 4 | 24 | max.effect. | No |
| T.H. | M | 60 | Right tibial neuralgia | middle | 2 | 48 | effective | No |
| O.H. | F | 73 | Spinal osteoporosis | middle | 4 | 48 | effective | No |
| O.H. | F | 33 | Lumbergo | middle | 6 | 48 | effective | No |
| K.T. | F | 82 | Fracture of of right femur | serious | 6 | 48 | effective | No |
| U.Y. | F | 28 | Left tibial neuralgia | middle | 5 | 72 | some-effect. | No |
| L.K. | F | 39 | Left tibial neuralgia | middle | 4 | 72 | ineffective | No |
| A.M | F | 49 | Left tibial neuralgia | serious | 4 | 48 | effective | No |
| M.H. | F | 47 | Deformed lumbar vertebra | middle | 4 | 24 | max.effect. | No |
| T.K. | M | 48 | Lumbergo | middle | 4 | 24 | max.effect. | No |
| T.K. | M | 48 | Both tibial neuralgia | middle | 4 | 24 | max.effect. | No |
| K.K. | M | 68 | Right shoulder articular circumferential Inflammation | middle | 5 | 48 | effective | No |
| M.K. | M | 57 | Right shoulder Joint contracture | serious | 4 | 24 | max.effect. | No |
| HM. | M | 62 | Left shoulder anesthesia | middle | 3 | 48 | effective | No |
| T.S. | M | 65 | Deformed lumbar vertebra | middle | 4 | 48 | effective | No |
| L K. | F | 70 | Right shoulder articular circumferential Inflammation | middle | 3 | 48 | effective | No |
| L M. | F | 56 | Both tibial neuralgia | serious | 6 | 72 | ineffective | No |
| S.Y. | F | 31 | Both tibial neuralgia | middle | 6 | 24 | max.effect. | No |
| K.Y. | F | 31 | Both tibial neuralgia | middle | 10 | 24 | max.effect. | No |
| S.H | F | 43 | Right shoulder articular circumferential inflammation | middle | 5 | 72 | ineffective | No |
| N M. | F | 40 | Left tibial neuralgia | middle | 6 | 24 | ineffective. | No |
| Y.K. | F | 38 | Right deformed Knee articular inflammation | middle | 3 | 48 | some -effect | No |
| M.T | F | 56 | Right tibial neuralgia | slight | 7 | 24 | max.effective. | No |
| Y.K. | M | 56 | Right shoulder articular circumferential inflammation | slight | 10 | 48 | max.effecftve | No |
| Y.K. | M | 56 | Deformed lumbar vertebra | middle | 5 | 48 | max.effective | No |
| I.E. | F | 59 | Both tibial neuralgia | middle | 8 | 24 | max.effect. | No |
| T.S. | F | 49 | Right tibial neuralgia | middle | 6 | 72 | max.effective. | No |
| Y.S | F | 63 | Both tibial neuralgia | slight | 12 | 24 | max.effect. | No |
| Y.S. | F | 63 | Left deformed Knee articular inflammation | middle | 10 | 24 | max.-effect. | No |

**TABLE 3**

| Name of patient | Sex distinction | Age | Name of disease | Condition of disease | Number of applications | Time of applications (Hr) | Judgement of results | Side effects |
|---|---|---|---|---|---|---|---|---|
| H.M. | F | 49 | Right tibial neuralgia | middle | 4 | 24 | max.effecdve. | No |
| H.K. | F | 74 | Right deformed hand articular inflammation | middle | 3 | 72 | some.-effect. | No |
| Y.N. | M | 64 | Lumbergo | middle | 5 | 48 | effective. | No |
| H.A. | F | 74 | Both deformed Knee articular inflammation | serious | 10 | 72 | max.effect. | No |
| S T. | M | 60 | Right upper arm inflammation | serious | 5 | 72 | effective | No |
| S.T. | M | 57 | Right upper arm cervical inflammation | serious | 5 | 72 | effective | No |
| T.H. | M | 61 | Ligament ossifying | serious | 10 | 72 | effective | No |
| M.H. | F | 46 | Right shoulder articular circumferential inflammation | middle | 6 | 24 | ,max.effect. | No |
| M.H. | F | 46 | Right upper arm Inflammation | middle | 2 | 24 | max.effect | No |
| M.I. | M | 69 | Deformed cervical vertebra disease | middle | 5 | 48 | max.effective | No |

**TABLE 4**

| Name of patient | Sex distinction | Age | Name of disease | Condition of disease | Number of applications | Time of applications (Hr) | Judgement of results | Side effects |
|---|---|---|---|---|---|---|---|---|
| F.S. | F | 63 | Lumbergo | middle | 4 | 24 | ineffective. | No |
| K.M. | F | 77 | Right shoulder neuralgia | middle | 4 | 48 | ineffective | No |
| M.Y. | F | 53 | Right shoulder neuralgia | middle | 5 | 24 | ineffective | No |
| O.I. | F | 55 | Left shoulder neuralgia | slight | 5 | 24 | ineffective | No |
| O.Y. | F | 38 | Left shoulder neuralgia | middle | 6 | 24 | ineffective | No |
| H.M. | F | 49 | Left shoulder neuralgia | middle | 4 | 72 | ineffective | No |
| Y.S. | F | 63 | Left shoulder neuralgia | serious | 6 | 24 | ineffective | No |
| M.T. | F | 56 | Left shoulder neuralgia | serious | 7 | 24 | ineffective | No |
| G.H. | F | 30 | Right shoulder neuralgia | middle | 4 | 24 | ineffective | No |
| T.H. | F | 72 | Right shoulder neuralgia | middle | 6 | 48 | ineffective | No |

Number of cases of examinees: 92 (Number being applied with the therapeutical device with a tape according to the present invention: 82, and Number of not being applied with a commutator: 10)

| Effects | Therapeutical device with a tape according to the present invention | No commutator (probe short-circuited) |
|---|---|---|
| (1) Max.effect. | 39 | 0 |
| (2) Effective | 33 | 0 |
| (3) Somewhat effective | 4 | 0 |
| (4) Ineffective | 6 | 10 |
| (5) Stop | 0 | 0 |
| (6) Aggravation | 0 | 0 |
| (7) Side effects | 0 | 0 |
| (8) Crucial side effects | 0 | 0 |

During the experiments, no stop and drop-out occurs, and the side effects judged as aggravation and danger have not been confirmed.

It is contemplated that the comparative device merely using a current collection probe 2 short-circuited by a copper wire and a tape 6 provides none of therapeutical effects. The reason why is that all the ten (10) cases a double blind test method used dividing to left and right of the experimental device and the comparative device, wherein one experimental device (the present intention) includes many effective cases while the other comparative device (not provided with a unidirectional current commutator) including no effective cases, that is, all of them are judged to be ineffective cases. Even the pressing observation in a touch therapy including an expression of patients, a difference therebetween apparently appears. It is convinced ofthat at the time of 10 experimental results for 11 persons, the comparison of results with the experimental device according to the present invention was now clarified. So, it is judged that there is no sense to apply the comparison device thereafter, stopping the operation.

As a result of totaling the experimental results as described above, it can be evaluated that the experimental device according to the present invention is a medical device which is greatly excellent in the effects with respect to the stiffness.

FIG. 2 is a perspective view showing a therapeutical device comprising a unidirectional current commutator having a protruding portion according to the present invention; FIG. 3 is a plan view as viewed from the top of FIG. 2, and FIG. 4 is a side view of a unidirectional current commutator having a protruding portion.

A protruding portion 5 is formed on the side in contact with the unidirectional current commutator 1, and when a therapeutical device B is viewed from the side, the protruding portion 5 is in the state protruded from a current collection probe 2.

As shown in FIGS. 2 to 4, the protruding portion 5 is formed on the side in contact with the unidirectional current commutator 1 whereby when the therapeutical device A is applied to the skin, the effect such as "acupuncture and moxa cautery" as "an application type contact needle" is produced to impart stimulation to the cell, thus improving the circulation of blood to enhance the therapeutical effect.

### EMBODIMENT 2

FIG. 7 is a side view of a unidirectional current commutator employing another form according to Embodiment 2 of the present invention.

A protruding portion 5 of a unidirectional current commutator 1 can be formed by a method of forming one or more constricted parts on suitable portions of the unidirectional commutator 1 formed in a cylindrical shape.

Also by the concavo-convex shape according to this method, the effect similar to that of Embodiment 1 is produced.

### EMBODIMENT 3

FIG. 6 is a plan view showing a therapeutical device with a tape having vent holes according to Embodiment 3 of the present invention.

A therapeutical device with a tape C according to Embodiment 1 comprises the therapeutical device B and the tape 6 applied to the skin as described in Embodiment 3. The tape 6 is formed circularly and has a tackiness only on one surface thereof, and the outer periphery of the tape surface is formed to be somewhat larger than that of the therapeutical device. A square vent hole 7 is provided in the center of the tape surface.

This vent hole 7 has the size to the extent that the unidirectional current commutator 1 is exposed. By the provision of the vent hole 7, the ion action and the potential action in the tissue of the unidirectional current commutator 1 are activated so that the commutation effect is enhanced, and itch and skin eruptions are hard to occur in the parts applied.

The method of applying the therapeutical device with a tape according to the present invention to the patient will be described below.

FIG. 7 is an explanatory view showing the state in which the therapeutical device according to the present invention is applied to the root between the thumb and the index finger of the hand of the human body.

FIG. 8 is an explanatory view showing the state in which the therapeutical device according to the present invention is applied to the back of an ankle of the human body.

When the therapeutical device according to the present invention is used, it is applied to suitable parts such as the neck, the shoulder, the back, the waist or the like by a tape 6 in the state in which a unidirectional current commutator 1, a current collection probe 2 and a protruding portion are placed in contact with the skin.

As one example of parts to which the device is applied, there can be mentioned the root between the thumb and the index finger of the hand as shown in FIG. 7 or the back of an ankle as shown in FIG. 8.

The totaled results with respect to 74 cases of the second experimental results in which the therapeutical device and the therapeutical device with a tape according to the present invention are given in Tables 5 and 6 below.

**TABLE 6**

| | | | | | |
|---|---|---|---|---|---|
| In the following table, | | | | | |
| M: male F: female | | | | | |

| Effect | Name | 1 | Age | Sex distinction | Name of diseases and pain parts |
|---|---|---|---|---|---|
| 1 | Sakamoto | A | 74 | F | (multiple articular rheumatism) .elbow joint pain |
| 1 | Nishikawa | T | 82 | M | (left shoulder pain).Left shoulder arthralgy |
| 1 | Nakayama | S | 68 | F | (multiple articular rheumatism) .both hands joint outside pain |
| 1 | Hyodo | N | 73 | M | (cerebral infarction sequella).right foot bottom pain |
| 1 | Yanai | T | 71 | F | (multiple articular rheumatism) .left knee joint pain |
| 1 | Ogawa | T | 59 | F | laft shoulder joint pain.left elbow pain |
| 1 | Mori | M | 61 | F | right shoulder pain.right toothache.right face.lumbago |
| 1 | Kajiyama | A | 66 | F | (deformed myelitis.osteoporosis).intercostal neuralgia |
| 1 | Kawazoe | Y | 64 | F | (recurrent rheumatism).left-lower leg pain.left knee joint pain |
| 1 | Nagai | T | 90 | F | right palm pain. right brown pain. |
| 1 | Inatsu | K | 65 | M | (right shoulder joint circumferential inflammation). right shoulder circumferential pain |
| 1 | Takahashi | S | 81 | F | (sequella of neck of femur fracture).lumbago.right lower leg pain |
| 2 | Usijima | M | 63 | F | (waist spinal stricture).lumbago.both lower legs pain |
| 2 | Ikezawa | S | 81 | F | (deformed myelitis).lumbago |
| 2 | Kamimura | S | 71 | F | (deformed myelitis.osteoporosis).lumbago |
| 2 | Ogawa | T | 59 | F | (articular rheumatism).shoulder neuralgia |
| 2 | Uchio | S | 64 | M | (cerebral infarction sequela.shoulder articulate inflamation).pain of right shoulder joint |
| 2 | likawa | K | 42 | F | shoulder neuralgia |
| 2 | Asami | N | 42 | F | intercostal nerves |
| 2 | Kawashima | T | 70 | F | back nerves |
| 2 | Kuwabara | S | 77 | F | shoulder nerves.right hand joint pain |
| 2 | Someno | Y | 43 | F | back nerves |
| 2 | Sakanishi | T | 49 | M | (deformed myelithis).shoulder nerves right knee joint pain |
| 2 | Shimoda | N | 54 | F | (deformed myelitis.osteoporosis).right knee joint pain |
| 2 | Sato | Y | 23 | F | shoulder nerves |
| 2 | Takahashi | Y | 62 | M | (intercostal nerves).lumbago |
| 2 | Tsuruta | T | 77 | F | (deformed myelitis.osteoporosis).lumbago.lower leg pain |
| 2 | Tashiro | Y | 45 | M | (whiplash sequella).shoulder pain.headache |
| 2 | Uemura | Y | 76 | F | (Both-side deformed articular disease).both articular disease |
| 2 | Tagami | A | 69 | F | (deformed myelitis. osteoporosis).lumbago.nervers |
| 2 | Tagami | T | 75 | F | (deformed lumbar vertebra.osteoporosis).lumbago |
| 2 | Nagaishi | H | 86 | F | (deformed myelitis).left sciatica |
| 2 | Nakayama | M | 84 | F | (deformed myelitis.sciatica. osteoporosis) left lower leg pain |
| 2 | Nojiri | K | 18 | M | shoulder nerves |
| 2/ | Hirakawa | S | 63 | F | (deformed lumbar vertebra.osteoporosis).lumbago |
| 2 | Hama | Y | 48 | F | shoulder nerves |
| 2 | Hama | R | 20 | F | shoulder nerves |
| 2 | Hama | Y | 48 | F | shoulder nerves |

**TABLE 7**

| Effect | Name | 1 | Age | Sex distinction | Name of diseases and pain parts |
|---|---|---|---|---|---|
| 2 | Hashimoto | N | 48 | F | shoulder nerves |
| 2 | Hayashi | J | 54 | F | shoulder nerves |
| 2 | Masuda | S | 68 | M | shoulder nerves |
| 2 | Morimoto | F | 42 | F | shoulder nerves |
| 2 | Morimoto | T | 16 | M | back nerves |
| 2 | Yamaguchi | S | 58 | F | (deformed lumbar vertebra.osteoporosis).left thigh pain |
| 2 | Yoshii | M | 72 | F | (both knee arthritis).shoulder pain. knee joint pain |
| 2 | Yamashita | H | 69 | M | (hernia.osteoporosis).lumbago |
| 2 | Yamashita | F | 64 | F | shoulder nerves |
| 2 | Wakamatsu | F | 74 | F | lumbago.thigh pain |
| 2 | Yamashita | M | 63 | M | both knee arthritis |
| 2 | Yamashita | Y | 59 | F | lumbago |
| 2 | Kanae | N | 76 | F | (rheumatism of joint).both lower leg pain |
| 2 | Ogawa | A | 19 | M | lumbago |
| 2 | Morimoto | F | 41 | F | shoulder pain.lumbago (30 - 50 min) |
| 2 | Hayashi | S | 87 | F | lumbago |
| 2 | Kawamoto | E | 64 | F | (deformed lumbar vertebra).lumbago |
| 2 | Nakajuna | M | 61 | F | (right shoulder joint inflammation).right shoulder joint pain |
| 2 | Ishida | S | 43 | M | (cervical spinal cord fracture and damage).shoulder pain |
| 2 | Nomoto | S | 56 | F | (knee articular disease). knee joint pain |
| 2 | Kiba | K | 60 | F | (knee articular disease). knee joint pain |
| 2 | Shimokawa | N | 33 | M | shoulder pain |
| 2 | Shimokawa | M | 27 | F | shoulder pain.lumbago |
| 2 | Kanzaki | M | 63 | M | (semicircular slope damage sequella).knee joint pain |
| 2 | Hashimoto | T | 70 | F | knee joint pain |
| 2 | Morinaka | H | 70 | F | [jimei]. shoulder pain |
| 2 | Maeda | S | 59 | M | right shoulder join inflamation |
| 2 | Toyosumi | K | 44 | M | lumbago |
| 2 | Tanabe | K | 68 | F | shoulder pain.lumbago |
| 3 | Ikeda | Y | 77 | F | (multiple joint rheumatism).both knee joint pain |
| 3 | Iwata | K | 65 | M | chronic hemicrania |
| 3 | Kamemoto | N | 79 | F | (deformed myelitis.osteoporosis).shoulder pain |
| 3 | To | N | 68 | M | hernia.back pain |
| 3 | Tanabe | M | 74 | M | (waist in-spinal stricture).lumbago.lower leg pain |
| 3 | Nagae | Y | 51 | F | intercostal nerves |
| 3 | Miyamoto | M | 64 | M | chronic shoulder nerves.(diabetes mellitus) |
| 3 | Noda | Y | 92 | F | (deformed myelitis.osteoporosis).lumbago |

**TABLE 8**

| | Age | Trouble parts | Date Injured | Date applied | Time | Effect |
|---|---|---|---|---|---|---|
| 1 | 20 | distortion of inside of left ankle | 7/3 | 7/10 | 2 hr | C |
| 2 | 24 | night knee joint pain | 7/12 | 7/13 | 30 min | A |
| 3 | 31 | swell of both front legs | | 7/13 | 1 day | B |
| 4 | 27 | swell of both front legs | | 7/15 | 1 day | A |
| 5 | 27 | swell of left thigh bicapitate muscle | | 7/17 | 1 day | B |
| 6 | 51 | pain of left ligament | | 7/18 | 1 hr | B |
| 7 | 27 | lumbago | 7/16 | 7/16 | 1 day | A |
| 8 | 27 | swell of left inner muscle | | 7/16 | 1 day | A |
| 9 | 24 | pain of left Achilles' tendon | | 7/23 | 1 day | B |
| 10 | 25 | pain of right Achilles' tendon | | 7/25 | 1 day | B |
| 11 | 31 | left knee pain | | 7/27 | 1 day | A |
| 12 | 24 | swell of both front legs | | 7/27 | 1 day | A |
| Effect: A - good B - somewhat good C - remains unchanged | | | | | | |

The above-totaled results are clinical data for which a physician conducted for patients whose diseases become quite worsened. It has been reported that despite the patients whose diseases become quite worsened, the effective rate approved by a doctor is not less than 90%, and the pain of patients of rheumatism being subjected to the scientific treatment was improved in a short period of time. As described above, it has been proved that the conspicuous effect (maximum effect) exhibited with respect to not only healthy persons but also patients. That is,
a. the "maximum effect" indicated by 1 noted in the column of Effect is for a person who obtains a pain-relieving effect within 15 to 24 minutes, and whose motion function is improved gradually,
b. the "effective" indicated by 2 noted in the column of Effect is for a person who considers a pain-relieving effect within 15 to 24 minutes, and
c. the "ineffective" indicated by 3 noted in the column of Effect is for a person who does no consider a pain-relieving effect within 15 to 24 minutes.

According to the above Tables 6 and 7, out of 74 cases, persons of the maximum effect are 12, persons of the effective are 8, and persons of the maximum effect plus the effective are 66, the effective rate being 90%.

The totaled results in which the therapeutical device and the therapeutical device with a tape according to the present invention are applied to 12 soccer players are shown in Table 8. This is referred to as the third experimental results.

According to Table 8, out of 12 persons, total of those (A: good) or (B: somewhat good) is 11 persons, and it has been confirmed that the effect exceeds 90%.

While in the foregoing, the respective embodiments of the present invention have been described, it is considered from the first to third experimental results that the present invention exhibits the particular therapeutical effect particularly for the stiffness of the body.

The concrete constitution of the present invention is not limited to the forms ofthe present embodiments, but the change in design without departing the scope of the present invention is included in the present invention.

For example, while in the above-described embodiments, a description has been made of the constitution in which a number of vent holes are formed in the surface of the tape, but the shapes and number of the tape holes are optional, and the case where it is formed of a material in which the whole tape surface has a permeability to air like gauze is also included in the present invention.

While in the above-described embodiments, a case has been explained in which a therapeutical device is directly applied to the skin, it is to be noted that the device can be applied to a supporter, a stomach band, an undershirt or the like for use.

Furthermore, while in the above-described embodiments, a constitution has been explained in which plating of metal of either gold, silver, titanium or platinum is applied to the surface of a current collection probe, it is to be noted that plating of other metal which is an electrically good conductor and takes to the skin can be used.

### INDUSTRIAL APPLICABILITY

As described above, according to the therapeutical device with a tape of the present invention, a current collection probe placed in conductive with both ends is provided around a unidirectional current commutator. Therefore, the device is applied hard to the affected part for a suitable continuous period of time whereby the acupuncture action is exhibited, and the circulation of blood can be placed in a proper condition beyond the disturbance of a living body current.

Further, the therapeutical device according to the present invention has, in addition to the above effects, the effect that the permeability to air is improved, the breath of the skin can be normalized, and even if the device is worn for a long period of time, no stink occurs.

Moreover, because ofthe improvement in permeability, the ion action and potential action in the tissue of a unidirectional current commutator become activated so that the commutation effect is enhanced and the affected part can be efficiently treated.

Furthermore, in the therapeutical device according to the present invention, the previous effect of the therapeutical device can be further enhanced by the provision of a number of pores.

Further, in the therapeutical device according to the present invention, the unidirectional current commutator has a protruding portion in contact with the skin. Therefore, the protruding portion can be placed in contact with the skin to thereby press the cell to effectively overcome stagnation of the circulation of blood.

In addition to the above effects, the therapeutical device according to the present invention has the constitution in which plating of either metal of gold, silver, titanium or platinum is applied to the surface of a current collection probe. Therefore, it is possible to protect the skin from harmful substances such as solder plating, and prevent the skin from skin eruptions caused by metal as well as to effectively collect a fine current generated in the body to enhance the commutation effect and therapeutical effect.

Further, in the therapeutical device having a protruding portion with a tape according to the present invention, the size of the vent holes of a tape is made to have the size to the extent that a part or the whole of a unidirectional current commutator is exposed. Therefore, it is possible to accumulate rays of light on the exposed unidirectional current commutator, to obtain the hot moxa canter effect since heat is generated in the therapeutical device, to activate a living body current due to the hot effect, and to enhance the commutation effect of the therapeutical effect.

Further, in the therapeutical device having a protruding portion with a tape according to the present invention, the tape is formed with a number of holes for the passage of air. Therefore, the permeability is improved, and even if the device is used for a long period of time, no stink occurs in the skin. Further, because of the improvement in permeability, the ion action and potential action in the tissue of a unidirectional current commutator become activated so that the commutation effect is enhanced and the affected part can be efficiently treated.

## Claims

1. A therapeutical device with an adhesive tape comprising:
a unidirectional current commutator (1), a current collection probe (2) and a tape (6), wherein said current collection probe (2) is formed from a copper wire extending from both polarity ends of said commutator (1) and being provided around said commutator (1) in a substantially circular shape in the periphery of an outer extending portion derived from both ends thereof, said wires (3) being wound around said commutator (1) by ¾ turn in a direction opposite to each other, said wires (3) having ends bent internally toward said unidirectional current commutator (1), wherein said current commutator (1) is located in the center and a current flows in said wires (3) therearound to form a magnetic flux, and the current collection probe (2) having its outer peripheral surface applied with a plating of one metal, which is an electrical good conductor, out of gold, titanium, platinum and silver, in which
said tape (6), having a tackiness on one surface thereof, is applicable hard to the skin surface of the affected part from one integrated surface in which the unidirectional current commutator (1) and the current collection probe (2) are integrated, so that the integrated surface is applicable to the skin surface, wherein a protruding portion (5) is provided on that side of said unidirectional current commutator (1) which is to be placed in contact with the skin surface and said tape (6) is provided with a vent hole (7) having the size to the extent that a part or the whole is exposed to the unidirectional current commutator (1) while being deviated from the surface thereof in a central portion of the tape (6) and is provided with a plurality of small holes (8) spaced apart from each other.

2. A therapeutical device according to Claim 1, wherein the plating of the outer peripheral surface of the current collection probe (2) creates no unusual change, such as skin eruptions resulting from contact for a long period of time on the skin surface.

## Patentansprüche

1. Therapeutische Vorrichtung mit einem Klebeband, umfassend:
eine eindirektionalen Stromkommutator (1), einen Stromsammelkopf bzw. - sensor (2) und ein Band (6), wobei der Stromsammelkopf bzw. -sensor (2) aus einem Kupferdraht gebildet ist, der sich von beiden Polaritätsenden des Kommutators (1) erstreckt und um den Kommutator (1) in einer im wesentlichen kreisförmigen Form in dem Umfang von einem äußeren, sich erstreckenden Bereich vorgesehen ist, der von beiden Enden davon abgeleitet ist, wobei die Drähte (3) um den Kommutator (1) um eine 3/4-Drehung in einer Richtung entgegengesetzt zueinander gewickelt sind, wobei die Drähte (3) Enden aufweisen, die nach innen zu dem eindirektionalen Stromkommutator (1) gebogen sind, wobei der Stromkommutator (1) im Zentrum angeordnet ist und ein Strom in den Drähten (3) darum fließt, um einen magnetischen Fluß auszubilden, und der Stromsammelkopf bzw. -sensor (2) seine äußere Umfangsoberfläche mit einer Plattierung aus einem Metall versehen aufweist, welches ein elektrischer, guter Leiter aus Gold, Titan, Platin und Silber ist, in welchem
das Band (6), welches eine Klebrigkeit auf einer Oberfläche desselben aufweist, schwer auf die Hautoberfläche des betroffenen Teils von einer integrierten Oberfläche aufbringbar ist, in welcher der eindirektionale Stromkommutator (1) und der Stromsammelkopf bzw. -sensor (2) integriert sind, so daß die integrierte Oberfläche auf die Hautoberfläche aufbringbar ist, wobei ein vorragender Abschnitt (5) an der Seite des eindirektionalen Stromkommutators (1) zur Verfügung gestellt ist, welcher in Kontakt mit der Hautoberfläche anzuordnen ist, und das Band (6) mit einem Luftloch (7) versehen ist, das die Größe in dem Ausmaß aufweist, daß ein Teil oder das Gesamte dem eindirektionalen Stromkommutator (1) ausgesetzt ist, während es von der Oberfläche davon in einem zentralen Bereich des Bands (6) abgeleitet bzw. abgelenkt ist und mit einer Mehrzahl von kleinen Löchern (8) versehen ist, die voneinander beabstandet sind.

2. Therapeutische Vorrichtung nach Anspruch 1, wobei die Plattierung der Außenumfangsoberfläche des Stromsammelkopfs bzw. -sensors (2) eine nicht übliche Änderung bewirkt, wie Hauterhebungen, die vom Kontakt für einen langen Zeitraum auf der Hautoberfläche resultieren.

## Revendications

1. Dispositif thérapeutique équipé d'une bande adhésive comprenant :
un commutateur de courant unidirectionnel (1), une sonde de collecte de courant (2) et une bande (6), dans lequel ladite sonde de collecte de courant (2) est formée d'un fil de cuivre s'étendant à partir des deux extrémités de polarité dudit commutateur (1) et étant placé autour dudit commutateur (1) selon une forme sensiblement circulaire à la périphérie d'une partie s'étendant vers l'extérieur dérivée des deux extrémités de celui-ci, lesdits fils (3) étant enroulés autour dudit commutateur (1) de 3/4 de tour dans une direction opposée l'un par rapport à l'autre, lesdits fils (3) ayant des extrémités courbées vers l'intérieur vers ledit commutateur de courant unidirectionnel (1), dans lequel ledit commutateur de courant (1) est situé au centre et un courant passe dans lesdits fils (3) afin de former un flux magnétique, et la sonde de collecte de courant (2) ayant sa surface périphérique externe recouverte d'un placage en un métal, qui est un bon conducteur électrique, parmi l'or, le titane, le platine et l'argent, dans lequel
ladite bande (6), ayant un pouvoir adhésif sur une surface de celle-ci, est applicable avec force sur la surface de la peau de la partie affectée depuis une surface intégrée dans laquelle sont intégrés le commutateur de courant unidirectionnel (1) et la sonde de collecte de courant, de telle sorte que la surface intégrée soit applicable sur la surface de la peau, dans lequel une partie faisant saillie (5) est située sur ce côté dudit commutateur de courant unidirectionnel (1) qui doit être placé en contact avec la surface de la peau et ladite bande (6) est pourvue d'un trou d'aération (7) ayant une taille telle qu'elle soit exposée en partie ou en totalité au commutateur de courant unidirectionnel (1), tout en étant déviée de la surface de celui-ci dans une partie centrale de la bande (6) et est pourvue d'une pluralité de petits trous (8) espacés les uns des autres.

2. Dispositif thérapeutique selon la revendication 1, dans lequel le placage de la surface périphérique externe de la sonde de collecte de courant (2) ne crée pas de changement inhabituel, telles que des éruptions cutanées suite à un contact pendant une période de temps prolongée avec la surface de la peau.
